# EUROPEAN PATENT SPECIFICATION

(11) **EP 3 941 543 B1**
(45) Date of publication and mention of the grant of the patent: **06.12.2023**
(21) Application number: 20719010.9
(22) Date of filing: 19.03.2020
(51) Int. Cl.: A61L 27/16, A61L 27/50, A61L 27/56

(54) **ORBITAL FLOOR TEMPLATE**
ORBITABODENSCHABLONE
GABARIT DE PLANCHER ORBITAL

(30) Priority: 19.03.2019 US 201962820320 P
(43) Date of publication of application: 26.01.2022
(73) Proprietor: Poriferous, LLC, Newnan, GA 30263 (US)
(72) Inventor: NOBLE, Aaron, Newnan, Georgia 30263 (US)
(74) Representative: Wright, Howard Hugh Burnby
(86) International application number: PCT/US2020/023604
(87) International publication number: WO 2020/191184

(56) References cited:
- US-A1- 2006 224 242

## Description

### BACKGROUND

During orbital or other craniofacial implant surgery, a surgeon may require a template in order to appropriately shape a final implant. In current practice, the surgeon typically pulls two final implants from stock. One will be the actual implant that will be positioned and remain implanted in the patient, and the second implant is used as a sizing template, even though this implant is of quality that it could also be positioned and remain in the patient. This is ultimately wasteful; a separate implant is used during surgery, but at implant cannot be charged for (either through insurance or to the patient).

US 2006/224242 A1 discloses an orbital floor implant and template kit, comprising: an orbital floor implant, and a template shaped to correspond to the orbital floor implant. The implant is porous and comprises high density polyethylene. The template can be cut with scissors or a scalpel.

### FIELD OF THE DISCLOSURE

The present disclosure relates generally to an orbital floor implant and template kit. The template is used during surgery and prevents the need to use a separate, new, sterile, separately-packaged actual implant for sizing purposes.

### BRIEF DESCRIPTION OF THE DRAWINGS

Figure 1 shows a perspective view of a patient's orbital floor with a template in use to size the implant.
Figure 2 shows a schematic of a template and an implant packaged together in sterile packaging.

### SUMMARY

The present disclosure thus provides an orbital floor implant and template kit as defined in claim 1. The two components are provided together in a single sterile package. This allows the surgeon to pull a single package and have the implant and a pre-sized template available during surgery. This avoids the need to pull a second implant from the operating room materials shelf for sizing purposes only. The scope of the invention is defined by the claims. Any references in the description to methods of treatment refer to the products of the present invention for use in a method for treatment.

### DETAILED DESCRIPTION

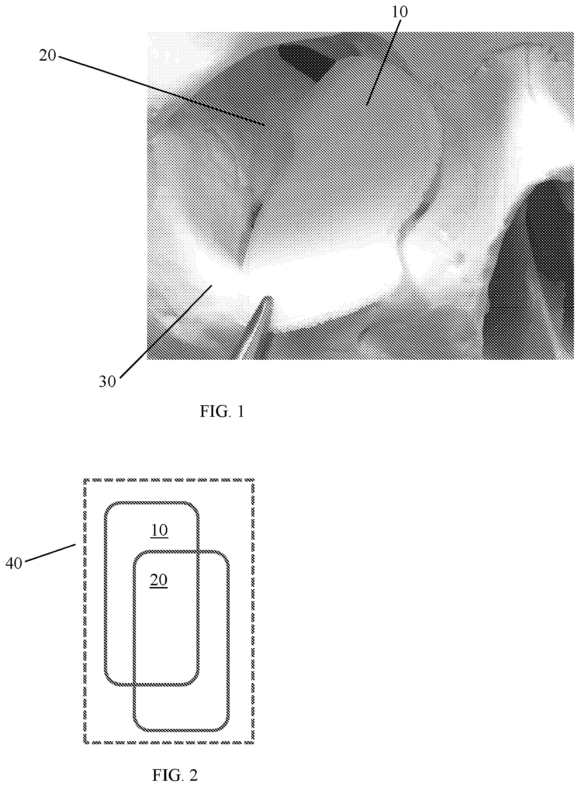

The accompanying figures illustrate the process for which the template may be used. Figure 1 is a close-up illustrating an implant 10 (shown as porous material) being sized by use of the disclosed template 20 (shown as a nonporous, thinner component, overlaid over the implant).

In some examples, the template 20 is a non-porous material. The template 20 is made of a material that can be bent and retain its shape. Various sizes of the template 20 may be provided along with variously sized implants 10. The templates 20 are closely sized to match the implants 10. The template 20 generally corresponds to the orbital floor 30 shape. It is provided with a curvature similar to a curvature of the implant. The template is provided of the same biocompatible materials of which the implant is made. Specifically, the template can be made from the same biocompatible HDPE (high density polyethylene) as the implant material. Rather than providing a template of a different material, the embodiments according to the invention use the same polymer material for the template that is used for the implant. This reduces risk of a non-implantable material being adversely introduced into the patient. For example, other templates have been made of silicone or other non-implant grade material. By contrast, using a polyethylene material for both the template and the implant can reduce risks or other complications. The template could be used as an implant itself as it is of the same implant grade material.

In one example, implants are typically about 0.85-1.0 mm thick. The disclosed template 20 be about 0.20 to 0.5 mm thick. In a specific example, disclosed template 20 may be about 0.4 mm thick. It is generally envisioned that the template 20 can be thinner than the implant 10, but this is not required. The template 20 is preferably designed so that it is able to be cut via a scalpel or surgical scissors. This can assist the surgeon during shaping and placement of the procedure.

A primary benefit of the disclosed template 20 is that it is included in a sterile pouch or packaging 40 with the implant 10, as shown by Figure 2. For example, if an implant is $100, it may be provided with a template, preventing the use of two implants at $200. This can save costs for hospitals, patients, and insurance companies, and came save time for surgeons. The template could be included at a nominal charge or not be charged at all.

## Claims

1. An orbital floor implant and template kit, comprising:
an orbital floor implant (10), and
a pre-sized template (20) that is a) shaped to correspond in size and shape to the orbital floor implant, b) has a curvature similar to the curvature of the orbital floor implant, and c) can be bent and retain the shape to which it has been bent,
wherein the pre-sized template and the orbital floor implant are made of the same polymeric material and are provided together in the same sterile package (40).

2. The kit of either of claim 1, wherein the template and the orbital floor template comprise high density polyethylene.

3. The kit of any of the preceding claims, wherein the template is made of nonporous material.

4. The kit of any of the preceding claims, wherein the implant is made of porous material.

5. The kit of any of the preceding claims, wherein the template can be cut with scissors or a scalpel.

6. The kit of any of the preceding claims, wherein the template is about 0.4 mm thick.

7. The kit of any of the preceding claims, wherein the template is made of a biocompatible material.

## Patentansprüche

1. Orbitalbodenimplantat- und Schablonenkit, umfassend:
- ein Orbitalboden-Implantat (10) und
- eine vordimensionierte Schablone (20), die a) so geformt ist, dass sie in Größe und Form dem Orbitalbodenimplantat entspricht, b) eine Krümmung aufweist, die der Krümmung des Orbitalbodenimplantats ähnlich ist, und c) gebogen werden kann und die Form beibehält, in die sie gebogen wurde,
- wobei die vordimensionierte Schablone und das Orbitalbodenimplantat aus demselben Polymermaterial hergestellt sind und zusammen in der gleichen sterilen Verpackung (40) geliefert werden.

2. Kit nach beiden des Anspruchs 1, wobei die Schablone und die Orbitalbodenschablone Polyethylen hoher Dichte enthalten.

3. Kit nach einem der vorhergehenden Ansprüche, wobei die Schablone aus einem nicht-porösen Material hergestellt ist.

4. Kit nach einem der vorhergehenden Ansprüche, wobei das Implantat aus porösem Material hergestellt ist.

5. Kit nach einem der vorhergehenden Ansprüche, wobei die Schablone mit einer Schere oder einem Skalpell geschnitten werden kann.

6. Kit nach einem der vorangehenden Ansprüche, wobei die Schablone etwa 0,4 mm dick ist.

7. Kit nach einem der vorhergehenden Ansprüche, wobei die Schablone aus einem biokompatiblen Material hergestellt ist.

## Revendications

1. Kit d'implant et de gabarit pour le plancher orbital comprenant :
- un implant de plancher orbital (10), et
- un gabarit prédimensionné (20) qui a) est mis en forme pour correspondre à la taille et à la forme de l'implant de plancher orbital, b) a une courbure similaire à la courbure de l'implant de plancher orbital et c) peut être courbé et retenir la forme selon laquelle il a été courbé,
- le gabarit prédimensionné et l'implant de plancher orbital étant réalisés dans la même matière polymère et fournis ensemble dans le même emballage stérile (40).

2. Kit selon la revendication 1,
dans lequel
le gabarit et le gabarit de plancher orbital sont en polyéthylène haute densité.

3. Kit selon l'une quelconque des revendications précédentes,
dans lequel
le gabarit est en une matière non poreuse.

4. Kit selon l'une quelconque des revendications précédentes,
dans lequel
l'implant est réalisé en une matière poreuse.

5. Kit selon l'une quelconque des revendications précédentes,
dans lequel
le gabarit peut être découpé avec des ciseaux ou un scalpel.

6. Kit selon l'une quelconque des revendications précédentes,
dans lequel
le gabarit a une épaisseur d'environ 0,4 mm.

7. Kit selon l'une quelconque des revendications précédentes,
dans lequel
le gabarit est en une matière biocompatible.
